# EUROPEAN PATENT SPECIFICATION

(11) **EP 2 227 714 B1**
(45) Date of publication and mention of the grant of the patent: **19.06.2013**
(21) Application number: 07865607.1
(22) Date of filing: 12.12.2007
(51) Int. Cl.: G02C 7/02, G02C 7/00, A61F 2/14

(54) **INTRACORNEAL LENSES HAVING A CENTRAL HOLE**
INTRACORNEALE LINSEN MIT EINEM ZENTRALEN LOCH
LENTILLE INTRACORNÉENNE PERCÉE D'UN TROU CENTRAL

(43) Date of publication of application: 15.09.2010
(73) Proprietor: Neoptics AG, 6331 Hünenberg (CH)
(72) Inventor: FEINGOLD, Vladimir, Laguna Niguel California 92644 (US); KOSMYNINE, Alexei, Aliso Viejo California 92656 (US)
(74) Representative: Hepp Wenger Ryffel AG
(86) International application number: PCT/US2007/087316
(87) International publication number: WO 2009/075685

(56) References cited:
- WO-A1-00/51526
- WO-A1-03/022168
- WO-A2-00/52516
- JP-A- 8 029 740
- US-A- 5 628 794
- US-A- 5 671 038
- US-A- 5 980 040

## Description

### BACKGROUND OF THE INVENTION

The present invention generally relates to intracorneal lenses, and to methods for correcting vision by insertion of an intracorneal lens in an eye of a patient.

It is known to provide an alternative to spectacles and extra-ocular contact lenses by using intraocular or intracorneal lenses for correcting deficiencies in visual acuity.

Intraocular lenses (IOLs) are typically provided for being inserted in the chamber of the eye, in the capsular bag or between the iris and the crystalline lens of the eye. Intraocular lenses typically comprise a central portion having optical corrective power, and a peripheral support portion. The support portion, known as a haptic, is generally provided to help manipulate the lens and also generally allows maintaining the lens in a given position within the eye.

US Patent Application Publication No. US 2004/0085511 A1 (Uno et al.) discloses an intraocular lens provided for being inserted in the posterior chamber of an eye. The lens has an optical portion and a support portion. When the lens is arranged in an eye, the edges of the support portion contact the outer edges of the posterior chamber, between the edges of the iris and the ciliary body. The support portion is dimensioned to maintain the optical portion properly aligned with the iris. The optical potion is dimensioned so that the opening of the iris never exceeds the diameter of the optical portion. The inside of an eye is filled with aqueous humors, and the lens comprises grooves and pores to allow the flow of the aqueous humor within the eye.

WO 2006/052279, from the present inventors, discloses an intraocular lens provided for being inserted in the posterior or anterior chamber of an eye. The lens has an optical portion and a support/haptic portion. The lenses arranged in the anterior chamber of an eye are held in position in the eye by the interaction of the haptic portion with the iridocorneal angle of the eye. The lenses arranged in the posterior chamber of an eye are held in position in the eye by the interaction of the haptic portion with the angle between the edges of the iris and the ciliary body of the eye. The lenses comprise grooves and pores to allow a flow of the aqueous humor within the eye. Further, the haptic portion of the lenses comprises orientation labels. The lenses can be inserted in the eye in a folded configuration, and unfolded within the eye. The orientation labels help the surgeon determining the position of the anterior and posterior faces of the lenses.

Intracorneal lenses differ in a number of aspects from the intraocular lenses. Intracorneal lenses are provided for being inserted within the cornea instead of within the chambers of the eye. Because intracorneal lenses are provided for being inserted within the cornea, they are smaller than intraocular lenses. Since intracorneal lenses and intraocular lenses have different positions with respect to the crystalline of the eye, an intracorneal lens and an intraocular lens must have different optical properties to correct a same abnormality of an eye.

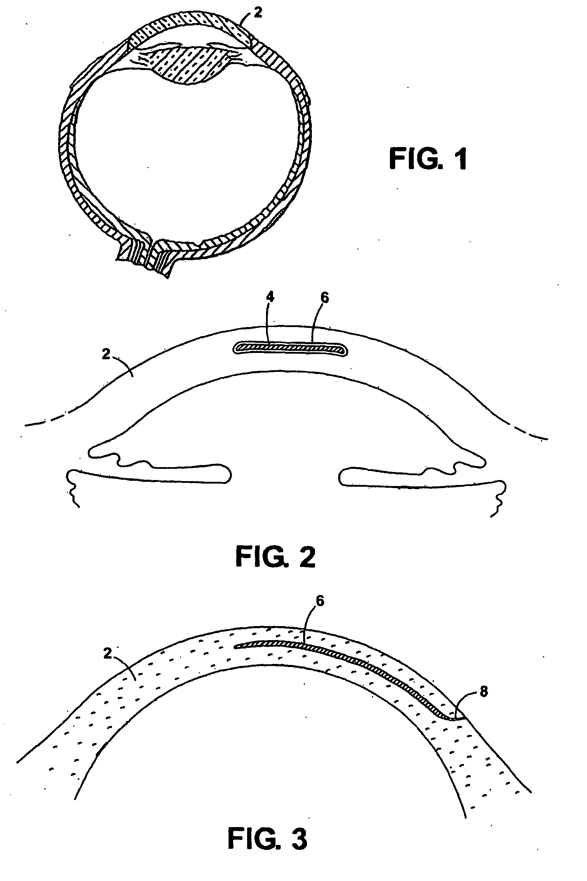

Figure 1 shows a cross section of an eye having a cornea 2. A variety of devices have been developed to prepare an opening in the cornea of an eye having visual abnormalities. An intracorneal lens is then inserted and maintained in the opening of the cornea, for example as shown in Figure 2. Figure 2 shows an intracorneal lens 4 in an opening 6 of a cornea 2 of an eye.

As detailed above, intraocular lenses have support portions that interact with the natural edges of the eye chambers to align the lenses with the eye. However, an intracorneal lens is inserted in a man-made opening in the cornea, which has no natural edges with which the lens could interact to align the lens with the eye.

It is nevertheless generally necessary to align precisely an intracorneal lens with a predetermined axis of the eye to obtain a desired correction of an abnormality of the eye.

WO 03/015674 to Feingold discloses a device provided for cutting in a cornea a pocket that is precisely positioned and dimensioned, and intracorneal lenses provided for being inserted in such pockets. In a preferred embodiment, the pocket is substantially circular with a lateral access opening smaller than a diameter of the pocket. Lenses are provided for having a diameter smaller than the diameter of the pocket outside of the cornea, and for swelling to the diameter of the pocket once in the cornea. This promotes retention of the lens in an aligned position in the cornea.

However, all intracorneal lenses may not be provided for swelling once introduced in the cornea. Further, cutting a pocket having precise position and dimensions can be difficult and/or time consuming.

Accordingly, there is a need for a device or a method that would allow implanting an intracorneal lens without having to use a lens provided for swelling once introduced in the cornea, or without having to cut a pocket of precise position and dimensions in the cornea.

US 5,628,794 is related to a specific intracorneal lens. The lens comprises a central opening in order to allow nutrient liquids to pass through the lens. The opening in the lens has a diameter of 0.1 mm.

WO 00/52516 describes lenses having a central opening, which should provide an optical effect, i.e. a pinhole effect impairing the optical properties of the lens.

### SUMMARY OF THE INVENTION

The present invention satisfies the above-noted need by providing a lens having a central hole with a size small enough to avoid impairing the optical properties of the lens, and big enough to allow the surgeon to see the hole and to align the hole with a mark showing an axis of the eye, when implanting the lens in the cornea.

In particular, the present invention provides for a lens provided for being implanted in a cornea according to claim 1.

According to an embodiment of the invention, the non-optical portion is surrounded by the optical portion.

According to an embodiment of the invention, the hole is a single hole.

According to an embodiment of the invention, the diameter of the hole varies along the depth of the hole.

According to an embodiment of the invention, a first portion of the walls of the hole follows a first portion of a cone, the diameter of the hole decreasing from a first outer diameter, at an entrance of the hole, to an inner diameter smaller than the first outer diameter, at an intermediate position within the hole, and a second portion of the walls of the hole follows a second portion of a cone, increasing from the inner diameter to a second outer diameter, at the other entrance of the hole.

According to an embodiment of the invention, a first portion of the walls of the hole follows a first portion of a torus center, the diameter of the hole decreasing from a first outer diameter, at an entrance of the hole, to an inner diameter smaller than the first outer diameter, at an intermediate position within the hole, and a second portion of the walls of the hole follows a second portion of a torus, increasing from the inner diameter to a second outer diameter, at the other entrance of the hole.

According to an embodiment of the invention, a first portion of the walls of the hole follows a portion of a cone, the diameter of the hole decreasing from a first outer diameter, at an entrance of the hole, to an inner diameter smaller than the first outer diameter, at an intermediate position within the hole, and wherein a second portion of the walls of the hole follows a portion of a torus, increasing from the inner diameter to a second outer diameter, at the other entrance of the hole.

According to an embodiment of the invention, a third portion of the walls of the hole, between the first and second portions of the walls of the hole, follows a cylinder having a diameter equal to the inner diameter.

According to an embodiment of the invention, the walls of the hole follow a cone from one entrance of the hole to the other entrance to the hole.

According to an embodiment of the invention, the walls of the hole follow a cylinder from one entrance of the hole to the other entrance of the hole.

According to an embodiment of the invention, each of the anterior and the posterior surfaces of the lens comprises at least a portion of one of the following surface types: spherical surface, with a single focus; spherical surface, with two or more focuses; non-spherical surface, with a progressive focus zone; toric surface; and flat surface.

According to an embodiment of the invention, at least one of the anterior and the posterior surfaces comprises a stepped portion.

### BRIEF DESCRIPTION OF THE DRAWINGS

Fig.1 is a cross-section of an eye.
Figure 2 is a sectional view of the anterior portion of an eye having an intracorneal lens disposed within the cornea of the eye.
Figure 3 is a cross-sectional view of a corneal pocket for receiving an intracorneal lens.
Figure 4a shows an elevation view of a lens according to an embodiment of the present invention.
Figure 4b shows a cross-section of the lens of Figure 4a.
Figure 4c is a close-up view of the center of Figure 4b (not according to the invention).
Figure 4d depicts a hole area of a particular embodiment.
Figure 5a illustrates a method according to the present invention.
Figure 5b is a top-view of an eye having a cornea with an intracorneal lens according to the present invention.
Figure 5c is a cross-section view of the cornea of Figure 5b.
Figure 5d is another cross-section view of the cornea of Figure 5b.
Figure 6a shows an elevation view of a lens according to another embodiment of the present invention.
Figure 6b shows a cross-section of the lens of Figure 6a.
Figure 6c is a close-up view of the center of Figure 6b (not according to the invention).
Figure 7a shows an elevation view of a lens according to another embodiment of the present invention.
Figure 7b shows a cross-section of the lens of Figure 7a.
Figure 7c is a close-up view of the center of Figure 7b (not according to the invention).
Figure 8a shows an elevation view of a lens according to another embodiment of the present invention.
Figure 8b shows a cross-section of the lens of Figure 8a.
Figure 8c is a close-up view of the center of Figure 8b (not according to the invention).
Figure 9a shows an elevation view of a lens according to another embodiment of the present invention.
Figure 9b shows a cross-section of the lens of Figure 9a.
Figure 9c is a close-up view of the center of Figure 9b (not according to the invention).
Figure 10a shows an elevation view of a lens according to another embodiment of the present invention.
Figure 10b shows a cross-section of the lens of Figure 10a.
Figure 10c is a close-up view of the center of Figure 10b (not according to the invention).
Figure 11a shows an elevation view of a lens according to another embodiment of the present invention.
Figure 11b shows a cross-section of the lens of Figure 11a.
Figure 11c is a close-up view of the center of Figure 11b (not according to the invention).
Figure 12a shows an elevation view of a lens according to another embodiment of the present invention.
Figure 12b shows a cross-section of the lens of Figure 12a.
Figure 12c is a close-up view of the edge of Figure 12b.
Figure 13a shows an elevation view of a lens according to another embodiment of the present invention.
Figure 13b shows a cross-section of the lens of Figure 13a.
Figure 13c is a close-up view of the edge of Figure 13b.
Figure 14 illustrates how light rays traverse an embodiment of the lens in Figures 12a-c.
Figure 15 illustrates how light rays traverse another embodiment of the lens in Figures 12a-c.
Figure 16a shows an elevation view of a lens according to another embodiment of the present invention.
Figure 16b shows a cross-section of the lens of Figure 16a and illustrates how light rays traverse the lens.
Figure 17a shows an elevation view of a lens according to another embodiment of the present invention.
Figure 17b shows a first cross-section of the lens of Figure 17a and illustrates how light rays traverse the cross-section of the lens.
Figure 17c shows a second cross-section of the lens of Figure 17a and illustrates how light rays traverse the cross-section of the lens.

### DETAILED DESCRIPTION

The present invention presents means to permanently, yet reversibly, correct defects of vision by disposing a lens in a pocket in a cornea. Various embodiments correct myopia, hyperopia, astigmatism, presbyopia, or a combination of these defects. It is to be understood that the present invention is not limited to treatment of these defects, and that treatment of other eye conditions is also within the scope of the invention. The correction may be permanent, if it remains satisfactory, and may also be reversed by removing the lens from the cornea.

The lenses according to the present invention are for example provided for being inserted in a corneal pocket as formed with the corneal pocket Keratome device disclosed in WO 03/015674 to Feingold. As detailed hereafter, the corneal pocket must be slightly larger than the lenses to let room to adjust the position of the lens within the pocket.

Figure 3 shows a cross-section of a cornea 2 wherein a pocket or opening 6 has been cut. An access opening 8 allows entering the opening 6.

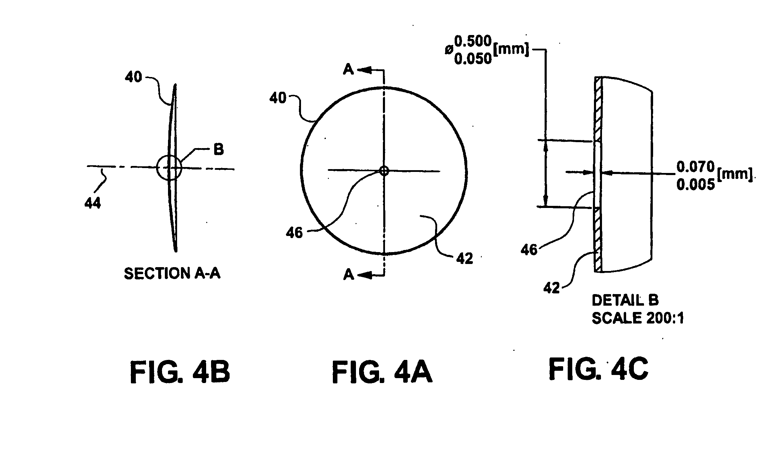

Figure 4a shows a refractive lens 40 according to an embodiment of the present invention. As detailed hereafter, lens 40 is provided to be inserted in a cornea opening such as shown in Figure 3. Lens 40 is a spherical lens, in which both the inner and outer surfaces are portions of a sphere, as shown in Figure 4b. Lens 40 is comprised of a single circular optical portion 42 having optical power. Optical portion 42 has an optical axis 44. As detailed hereafter, in some embodiments of the invention the lens can comprise a non-optical portion, concentric or not with the axis 44, within or around the optical portion. The lens can have a diameter of between 1.5 and 6 millimeter.

The lens 42 further comprises a hole 46 that is concentric with the optical axis 44 of the lens and that goes though the lens 40. According to the invention, the dimension and shape of the hole are chosen so that the hole does not impair the optical properties of the lens, and remains visible to one (such as a surgeon) that manipulates the lens. The hole has a diameter comprised between 100 and 200 micrometer. Preferably, the hole has a diameter of 150 micrometer. The inventors have found that, surprisingly, a hole having the preferred dimensions does not to impair the optical properties of the lens (is not noticed by the patient), and at the same time remains visible to a surgeon that manipulates the lens. This discovery was counter intuitive because one would think that a hole big enough to be seen by the surgeon would have to be so big that it would impair the optical properties of the lens, for example by inducing edge glare from the edge of the hole. However, this is not the case with the preferred dimensions of the hole. For the present invention, it is considered that if the hole does not induce a significant glare that can be noticed by a user having an eye bearing the lens, the hole does not impair the optical properties of the lens.

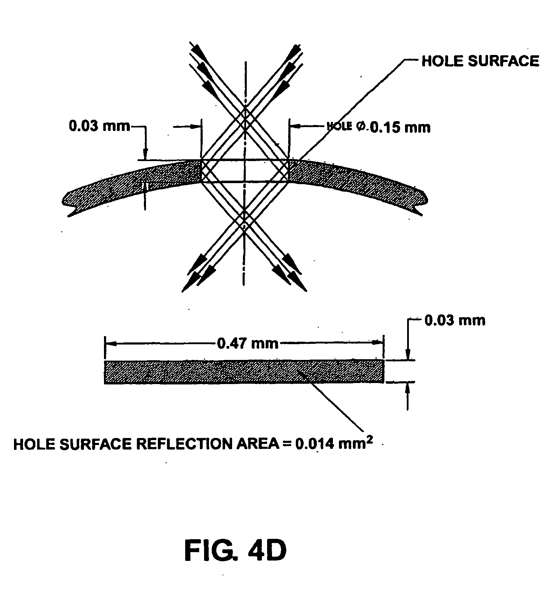

As shown in Fig. 4c (hole diameter not according to the invention), the walls of the hole can follow a cylinder from one entrance of the hole to the other entrance of the hole. In order to reduce the glare induced by the hole, the size and shape of the hole are preferably chosen to minimize the surface reflection area of the hole. Figure 4d shows for example that for a thickness of the lens around the hole of 0.03mm and a hole having a diameter of 150 micrometer, the hole surface area is of 0.014 square millimeter. The thickness of the lens around the hole can be comprised between 0.07 millimeter and 0.005 millimeter. The inventors have found that such a hole surface area does not generate a glare that is noticed by a user having an eye bearing the lens.

As detailed hereafter, the walls of the hole can also be different from a simple cylinder to reduce further the surface reflection area of the hole.

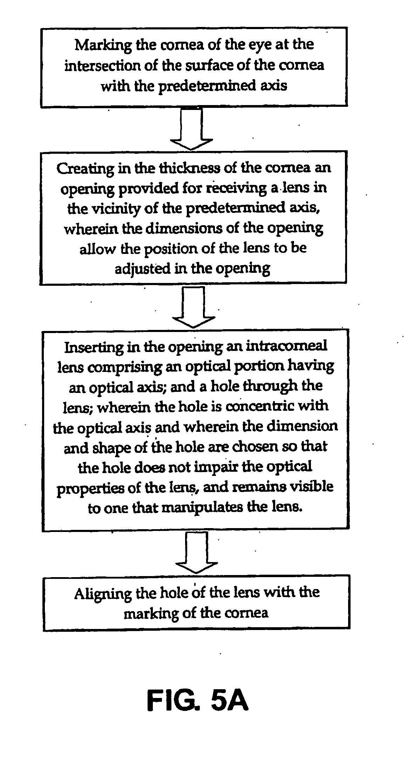

A lens according to the present invention allows implementing a method of correcting optical properties of a cornea of an eye along a predetermined axis of the eye. Such method is for example illustrated in Figure 5a.

In step 1, one marks the cornea of the eye at the intersection of the surface of the cornea and of a predetermined axis along which the optical properties of the cornea should be corrected. The marking can be made on the external surface of the cornea using a laser, a sharp and/or pointed device, by using pigmentation or by letting a marker device be temporarily pinned or adhered to the surface of the cornea.

In step 2, one creates in the thickness of the cornea an opening provided for receiving a lens, such as the opening shown in Figure 3. The opening can be created with a corneal pocket keratome as disclosed in WO 03/015674; or using a laser. The laser may be used and guided under computer control, as is well known in the art. A corneal opening may be formed by methods similar to those used during LASIK (laser-assisted in-situ keratomileusis) procedures. Alternatively, a corneal pocket can be formed using a laser and a mask that shapes the pocket, as disclosed in WO2008/072092 to Feingold. Alternatively, a corneal pocket may be formed manually by the surgeon using hand-held instruments.

A corneal flap (not shown) can be formed as an alternative to a corneal opening.

The dimensions of the opening must be such that they allow the position of the lens to be adjusted in the opening. The depth at which the opening is made under the external surface of cornea is chosen with regards to what must be corrected in the optical properties of the cornea, to the type of lens to be used, etc... The order of step 1 and 2 can be inverted if appropriate.

In step 3, one inserts in the opening a lens according to the invention, with an optical portion having an optical axis and a hole through the lens, wherein the hole is concentric with the optical axis and wherein the dimension and shape of the hole are chosen so that the hole does not impair the optical properties of the lens, and remains visible to one that manipulates the lens. The lens is provided for correcting the optical properties of the cornea when inserted in the opening and with the axis of the lens aligned on the predetermined axis of the eye. The lens and the opening are such that centering the hole on the marking of the cornea aligns the axis of the lens on the predetermined axis of the eye. A fluid can be inserted in the opening for easing the introduction of the lens. A canula or a small spatula can be used to move the lens to its desired location.

Then, in step 4, one aligns the hole of the lens with the marking of the cornea. The dimensions of the hole are such that the hole is still visible to one that manipulates the lens through the part of the cornea above the opening where the lens is. The inventors have noted that if the diameter of the hole is too large, it may become difficult to align precisely the hole with the marking of the cornea. This is for example because the edges of the hole become too remote from the marking to know if they are equally distant from the marking. Also for this reason, the diameter of the hole is preferably of the dimensions detailed above.

The opening of the cornea is self sealing and after a few days, the epithelium covers the access of the opening.

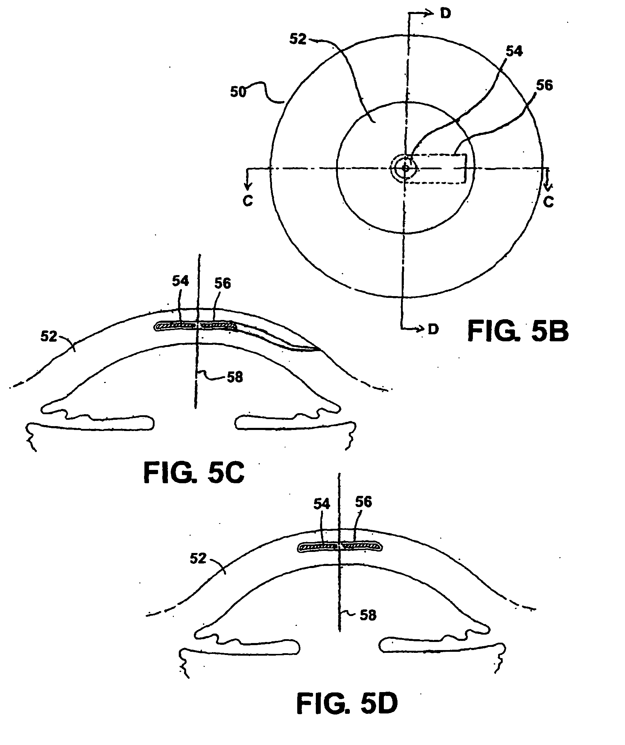

Figure 5b show a top-view of an eye 50 having a cornea 52 with an intracorneal lens 54 according to the present invention in a corneal opening 56.

Figure 5c is a cross-section view of the cornea of Figure 5b along a plane C-C including the predetermined axis 58 of the eye; and Figure 5d is a cross-section view of the cornea of Figure 5b along a plane D-D also including the predetermined axis 58 of the eye and perpendicular to plane C-C. The predetermined axis of the eye can be centered or not with respect to the cornea, depending on the abnormality to be corrected.

It is known that the cells of the cornea receive nutrients via diffusion from the tear fluid at the outside and the aqueous humour at the inside and also from neurotrophins supplied by nerve fibres that innervate it. Oxygen is received through the air. It is known to form intracorneal lenses of a biocompatible material that permits sufficient gas diffusion to allow adequate oxygenation of internal eye tissues (such materials may include silicone, hydrogels, urethanes or acrylics). However, the inventors have noticed that, when an intracorneal lens is implanted in a cornea, providing a hole according to the present invention in the lens seems to enhance the transfer of the nutrients within the cornea, which is beneficial to the cornea and for example eases the healing of the cornea after implantation of the lens. Furthermore, the inventors have noted that by providing a flow through the hole, no haze or cloudiness can be observed in the cornea after a healing period.

Advantageously, the hole of a lens according to the invention passes though the center of the lens. The inventors have noticed that, in particular when the lens has the general shape of a dome, with a concave surface and a convex surface, arranging the hole in the center of the lens seems to enhance further the transfer of the nutrients within the cornea, which is even more beneficial to the cornea.

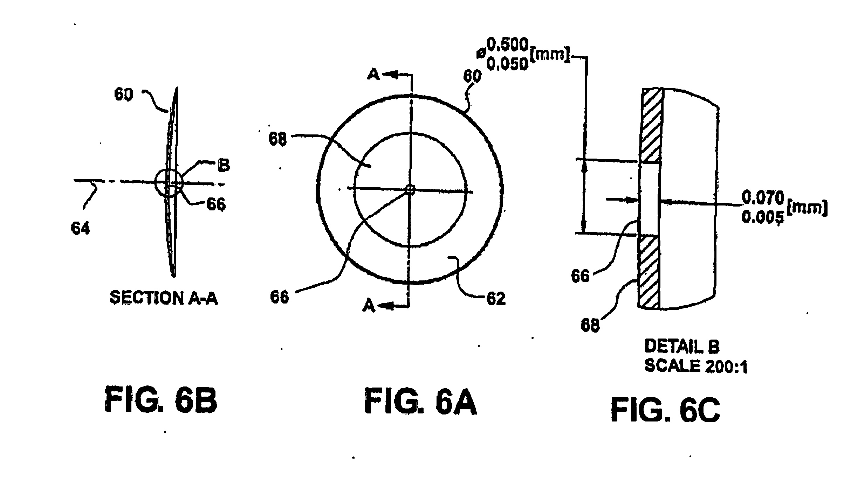

Figure 6a shows a lens 60 according to another embodiment of the present invention. Lens 60 is a spherical lens, in which both the inner and outer surfaces are portions of a sphere, as illustrated in Figure 6b. Lens 60 comprises a circular optical portion 62 with an optical axis 64 and a hole 66 coaxial with the axis 64. The lens 60 also comprises a circular non-optical portion 68 having no optical power, within the optical portion 62 and concentric with the optical portion 62.

In some other embodiments of the invention, the positions of the optical portion (such as optical portion 62 in the embodiment of Figure 6a) and the non-optical portion (such as non-optical portion 68 in the embodiment of Figure 6a) can be inverted.

Some other embodiments of the invention can comprise a number of concentric optical and non-optical portions alternated in any manner (1-1, 1-2, 2-1, etc...).

In some other embodiments of the invention, the non-optical portion can be non-concentric with the axis of the optical portion.

As shown in Fig. 6c (hole diameter not according to the invention), the walls of the hole can follow a cylinder from one entrance of the hole to the other entrance to the hole. However, as detailed hereafter, the walls of the hole can also be shaped otherwise to reduce the risk of creating a glare on the walls of the hole.

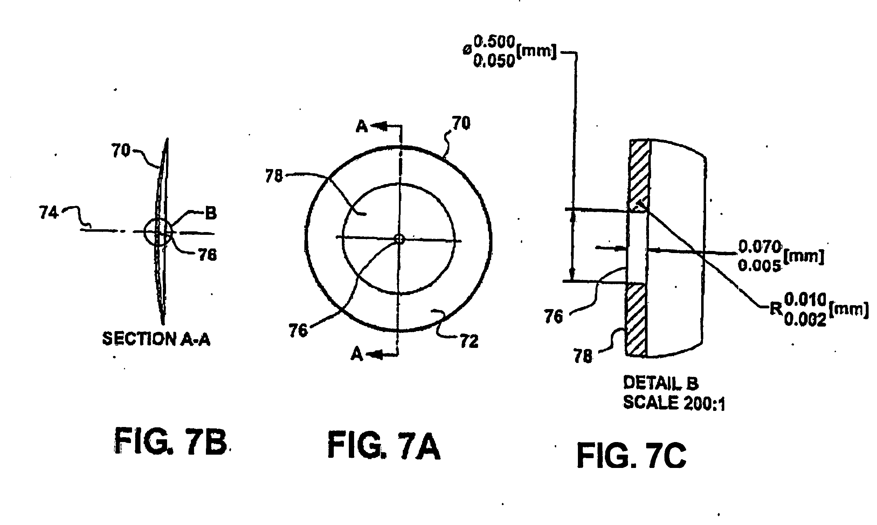

Figure 7a shows a lens 70 according to another embodiment of the present invention. Lens 70 is a spherical lens, in which both the inner and outer surfaces are portions of a sphere, as illustrated in Figure 7b. Lens 70 comprises a circular optical portion 72 with an optical axis 74 and a hole 76 coaxial with the axis 74. The lens 70 also comprises a circular non-optical portion 78 having no optical power, within the optical portion 72 and concentric with the optical portion 72.

As shown in Fig. 7c (hole diameter not according to the invention), the diameter of the hole 76 varies along the depth of the hole. A first portion of the walls of the hole follows a first portion of a torus: the diameter of the hole decreases from a first outer diameter, at an entrance of the hole, to an inner diameter smaller than the first outer diameter, at an intermediate position within the hole. A second portion of the walls of the hole follows a second portion of a torus, increasing from the inner diameter to a second outer diameter, at the other entrance of the hole. The radius of curvature of the revolved circle of the torus can be comprised between 0.01 millimeter and 0.002 millimeter. A third portion of the walls of the hole, between the first and second portions of the walls of the hole, is cylindrical and has a diameter equal to the inner diameter. In figure 7c, the first and second outer diameters are shown equal. However, they can also be different.

The lens shown in Figures 7a-c is spherical. However, as detailed hereafter, a lens according to the present invention can as well be aspheric.

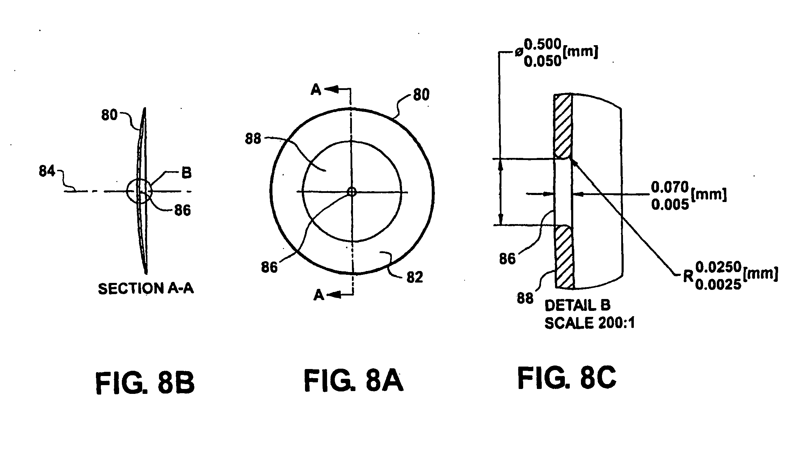

Figure 8a shows a lens 80 according to another embodiment of the present invention. Lens 80 is a spherical lens, in which both the inner and outer surfaces are portions of a sphere, as illustrated in Figure 8b. Lens 80 comprises a circular optical portion 82 with an optical axis 84 and a hole 86 coaxial with the axis 84. The lens 80 also comprises a circular non-optical portion 88 having no optical power, within the optical portion 82 and concentric with the optical portion 82.

As shown in Fig. 8c (hole diameter not according to the invention), the diameter of the hole varies along the depth of the hole. A first portion of the walls of the hole follows a first portion of a torus: the diameter of the hole decreases from a first outer diameter, at an entrance of the hole, to an inner diameter smaller than the first outer diameter, at an intermediate position within the hole. A second portion of the walls of the hole follows a second portion of a torus, increasing from the inner diameter to a second outer diameter, at the other entrance of the hole. The radius of curvature of the revolved circle of the torus can be comprised between 0.025 millimeter and 0.0025 millimeter. In figure 8c, the first and second outer diameters are shown equal. However, they can also be different.

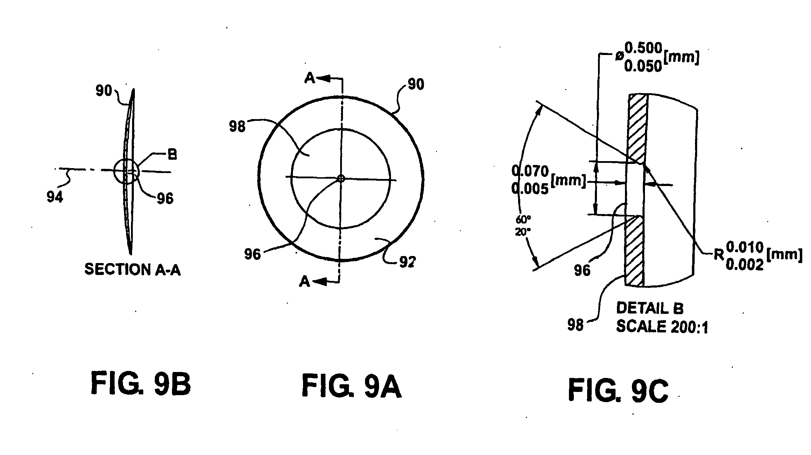

Figure 9a shows a lens 90 according to another embodiment of the present invention. Lens 90 is a spherical lens, in which both the inner and outer surfaces are portions of a sphere, as illustrated in Figure 9b. Lens 90 comprises a circular optical portion 92 with an optical axis 94 and a hole 96 coaxial with the axis 94. The lens 90 also comprises a circular non-optical portion 98 having no optical power, within the optical portion 92 and concentric with the optical portion 92.

As shown in Fig. 9c (hole diameter not according to the invention), the diameter of the hole varies along the depth of the hole. A first portion of the walls of the hole follows a portion of a cone, the diameter of the hole decreasing from a first outer diameter, at an entrance of the hole on the anterior face of the lens, to an inner diameter smaller than the first outer diameter, at an intermediate position within the hole. A second portion of the walls of the hole follows a portion of a torus, increasing from the inner diameter to a second outer diameter, at the other entrance of the hole on the posterior face of the lens. The portion of cone followed by the hole walls can belong to a cone formed by rotating a triangle having an angle of 10 to 30 degrees around the axis of the hole.

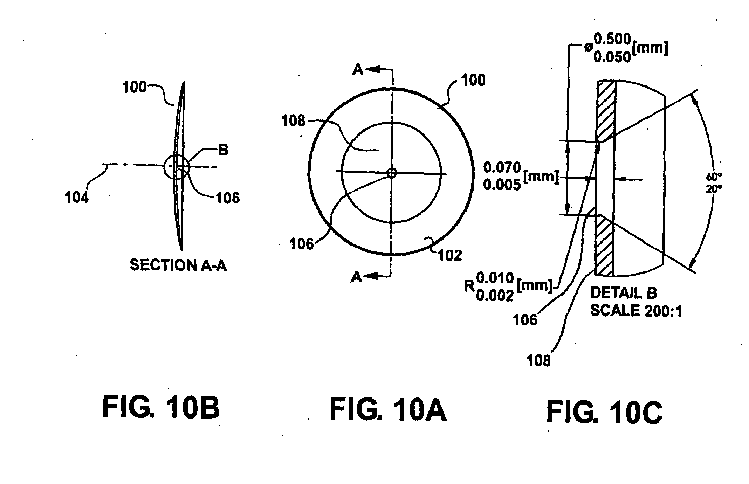

Figure 10a shows a lens 100 according to another embodiment of the present invention. Lens 100 is a spherical lens, in which both the inner and outer surfaces are portions of a sphere, as illustrated in Figure 10b. Lens 100 comprises a circular optical portion 102 with an optical axis 104 and a hole 106 coaxial with the axis 104. The lens 100 also comprises a circular non-optical portion 108 having no optical power, within the optical portion 102 and concentric with the optical portion 102.

As shown in Fig. 10c (hole diameter not according to the invention), the diameter of the hole varies along the depth of the hole. A first portion of the walls of the hole follows a portion of a cone, the diameter of the hole decreasing from a first outer diameter, at an entrance of the hole on the posterior face of the lens, to an inner diameter smaller than the first outer diameter, at an intermediate position within the hole. A second portion of the walls of the hole follows a portion of a torus, increasing from the inner diameter to a second outer diameter, at the other entrance of the hole on the anterior face of the lens.

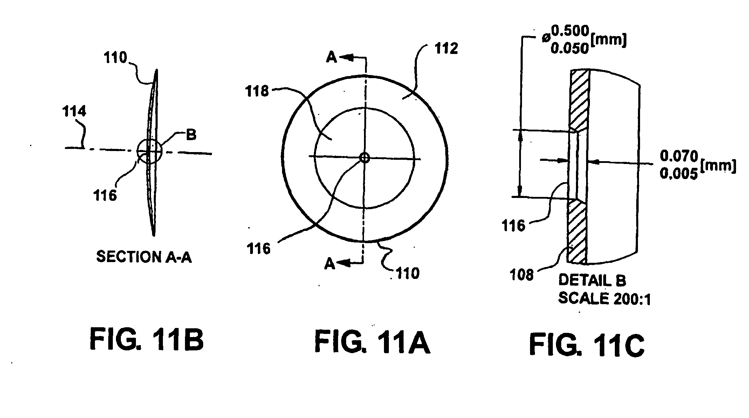

Figure 11a shows a lens 110 according to another embodiment of the present invention. Lens 110 is a spherical lens, in which both the inner and outer surfaces are portions of a sphere, as illustrated in Figure 11b. Lens 110 comprises a circular optical portion 112 with an optical axis 114 and a hole 116 coaxial with the axis 114. The lens 110 also comprises a circular non-optical portion 118 having no optical power, within the optical portion 112 and concentric with the optical portion 112.

As shown in Fig. 11c (hole diameter not according to the invention), the diameter of the hole varies along the depth of the hole. A first portion of the walls of the hole follows a first portion of a cone, the diameter of the hole decreasing from a first outer diameter, at an entrance of the hole, to an inner diameter smaller than the first outer diameter, at an intermediate position within the hole. A second portion of the walls of the hole follows a second portion of a cone, increasing from the inner diameter to a second outer diameter, at the other entrance of the hole. In figure 11c, the first and second outer diameters are shown equal. However, they can also be different.

According to an embodiment (not illustrated), the walls of the hole can follow a cone from one entrance of the hole to the other entrance to the hole.

The lenses shown in Figures 4a-c and 6a-c to 11a-c are all refractive spherical lenses in which both the inner and outer surfaces are portions of a sphere. However, the present invention is not limited to such lenses. For example, a lens according to the present invention can be a diffractive lens, for example a multi-step lens, and include an annular series of lens sections between the outer edge of the lens and the central portion of the lens. The greater range and control of refraction permitted by a multi-step lens is particularly useful for correction of presbyopia by the method and apparatus of the present invention. The annular ridges of the multi-step lens will resist lateral displacement, but a multi-step lens may also be given retention features. A multi-step lens can have an outer surface (anterior or posterior) that is a portion of a sphere, while the other outer surface is comprised of a series of annular sections of lenses of decreasing size.

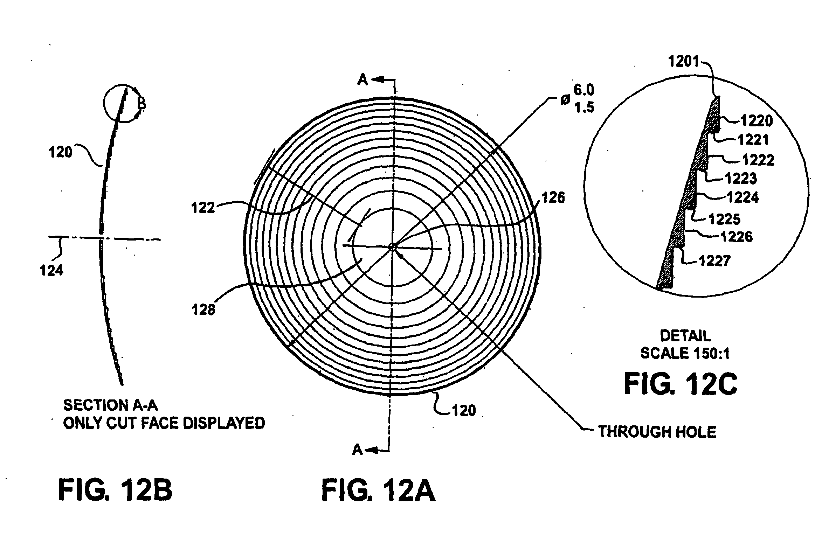

Figure 12a shows a reduced thickness, multi-step lens 120 according to an embodiment of the present invention. The outer surface of lens 120 is a portion of a sphere, as illustrated in Figure 12b. Lens 120 comprises a circular optical portion 122 with an optical axis 124 and a hole 126 coaxial with the axis 124. The lens 120 also comprises a circular non-optical portion 128 having no optical power, within the optical portion 122 and concentric with the optical portion 122. As detailed in Figure 12c, optical portion 122 is comprised of a series of concentric circular rings 1220, 1222, 1224, 1226, etc... formed on the inner surface of the lens in a stepped arrangement. In the embodiment shown in Figure 12c, the concentric rings 1220, 1222, 1224, 1226, etc... follow each a plane perpendicular to the axis 124. Further, the concentric rings 1220, 1222, 1224, 1226, etc...are connected to each other by walls 1221, 1223, 1225, 1227, etc... that follow each a cylinder having the same axis as the lens. The junctions between the rings 1220, 1222, 1224, 1226, etc... and the cylindrical walls 1221, 1223, 1225, 1227, etc... can be rounded. The external edge of the lens 120 can for example be beveled and follow a portion of a cone 1201 that is concentric with axis 124.

The shape of hole 126 is not shown in Figures 12a-c. However, the hole of a lens according to the present invention can have any of the shapes shown in the previous figures or any other appropriate shape.

The number and size of the rings 1220, 1222, 1224, 1226, etc... shown in Figures 12a-c is only given as an example. Any appropriate number and size can be used. Further, each ring is shown following parallel planes, but if appropriate each ring or some of the rings can follow a plane not parallel with the others, or a portion of a cone, of a sphere, of a tore, of an elliptic, parabolic or hyperbolic surface or of a polyhedron (having flat or non flat surfaces).

Also, the rings 1220, 1222, 1224, 1226, etc... are shown circular and concentric, but if appropriate they can have each a different shape and be for example elliptic or have different centers.

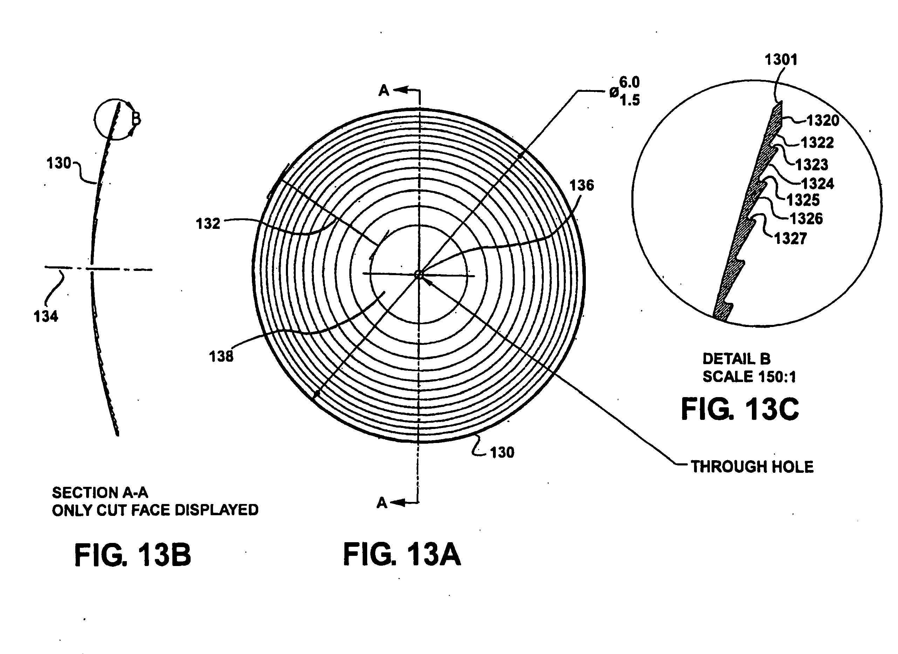

Figure 13a shows a reduced thickness, multi-step lens 130 according to another embodiment of the present invention. The outer surface of lens 130 is a portion of a sphere, as illustrated in Figure 13b. Lens 130 comprises a circular optical portion 132 with an optical axis 134 and a hole 136 coaxial with the axis 134. The lens 130 also comprises a circular non-optical portion 138 having no optical power, within the optical portion 132 and concentric with the optical portion 132. As detailed in Figure 13c, optical portion 132 is comprised of a series of concentric portions of cones of decreasing size 1322, 1324, 1326, etc... formed on the inner surface of the lens in a stepped arrangement and having each the same axis as the axis 134 of the lens. In the embodiment shown in Figure 13c, the portion of cones 1322, 1324, 1326, etc......are connected to each other by walls 1323, 1325, 1327, etc... following each a cylinder having the same axis as the lens. The junctions between the portion of cones 1322, 1324, 1326, etc... and the cylindrical walls 1323, 1325, 1327, etc... can be rounded. In Figure 13c, a plane ring 1320 connects the edge of the lens and the external-most edge of the portion of cone 1322. The external edge of the lens 130 can for example be beveled and follow a portion of a cone 1301 that is concentric with axis 134.

According to some embodiments of the invention, the portion of cones can alternatively be portions of spheres or portions of toric, elliptic, parabolic or hyperbolic surfaces. Alternatively, each portion of cone can be replaced by a series of portions of cone having different angles. The number and size of the cones shown in the Figures is only given as an example. Any appropriate number and size can be used.

Figures 12a-c and 13a-c show lenses having an anterior surface that is a portion of a sphere, and a stepped posterior surface. However, a lens according to the present invention can alternatively have a posterior surface that is a portion of a sphere, and a stepped anterior surface. A lens according to the present invention can also alternatively have stepped anterior and posterior surfaces.

A lens according to the present invention can have a single focal length. Such lenses are generally sufficient to correct simple myopia or hyperopia.

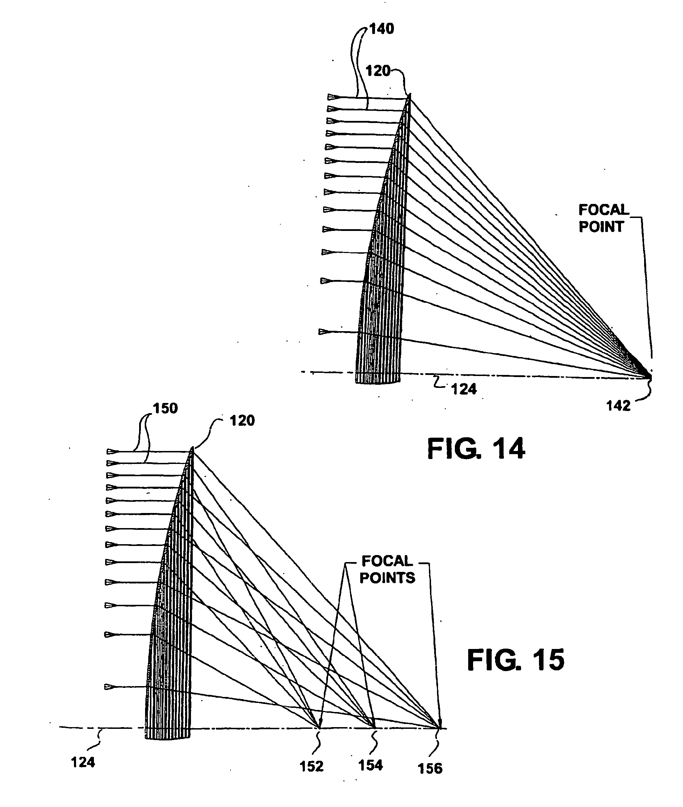

Figure 14 illustrates how light rays 140 traverse the upper portion of a cross-section of a lens 120 such as shown in Figures 12a-c in an embodiment where the lens is a lens having a single focal point 142.

However, lenses having variations in either refractive index or lens shape, or both, may be used advantageously as part of the present invention to establish a multifocal lens. The focal length of such lens is not constant, but varies across the expanse of the lens. Such lens multifocality can be used to compensate for presbyopia, by causing one portion of the light incoming to the eye to be focused if the source is far away, while another portion of the fight is focused when the source is close (as when reading). The effectiveness of such varying focal length lenses relies upon reliable positioning of the lens, as is provided by the present invention, in order to avoid misalignment of the lens, and to simplify adaptation to a plurality of focal lengths by the visual processing facilities. For example, presbyopia may be compensated by situating a small area, for example less than 3 mm diameter, of focal-length reducing lens at the center of the cornea. Such location will have greater effect in high-light conditions (as are typical for reading), when the pupil is small, and proportionally less effect under lower lighting conditions, such as night driving, when the pupil is large. Thus the lens location with respect to the pupil must be maintained; and the brain will adapt more easily to a non-uniform focus of the eye which is at least constant:

Figure 15 illustrates how light rays 150 traverse the upper portion of a cross-section of a lens 120 such as shown in Figures 12a-c in an embodiment where the lens is a lens having three focal points 152, 154 and 156.

According to an embodiment of the invention, multifocality may also be accomplished using a non multi-step lens having non-spherical surfaces.

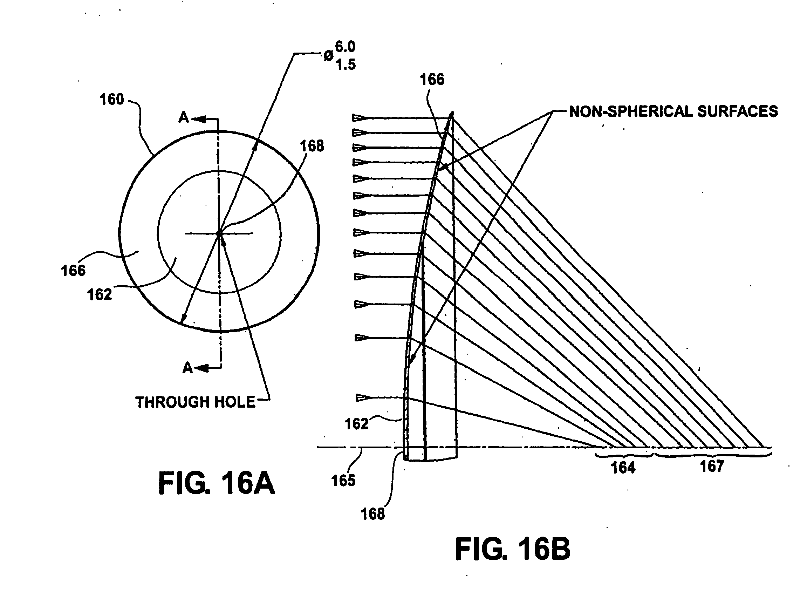

Figure 16a shows an elevation view of a non multi-step lens having non-spherical surfaces. A cross section of the upper part of such lens is shown in Figure 16b. The lens 160 of Figures 16a-b comprises a central non-spherical dome portion 162 that defines a first focal zone 164 along the axis 165 of the lens. The central portion 162 is surrounded by a peripheral non-spherical annular portion 166 that defines a second focal zone 167 along the axis 166 of the lens. A hole 168 according to the present invention traverses the center of the lens. Non-spherical surfaces may be such that a cross-section of the surfaces along the axis of the lens follows a portion of an ellipse, a parabola or a hyperbola.

According to an embodiment of the invention, varying focal length of toric surfaces of the lens can be used to correct astigmatism. Lenses according to the present invention can be multifocal lenses that simultaneously correct or compensate various combinations of defects including myopia, hyperopia, astigmatism and presbyopia.

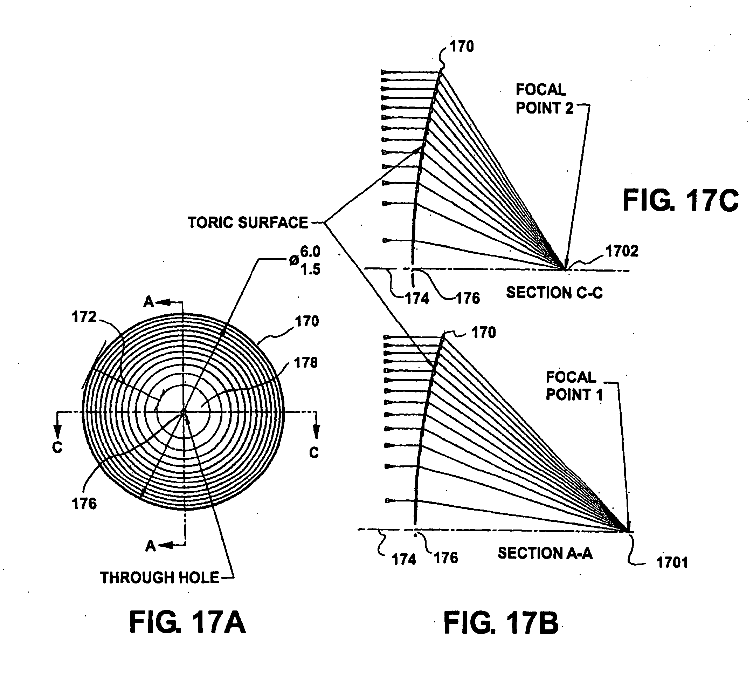

Figure 17a shows such a lens 170 according to the present invention. The anterior outer surface of lens 170 follows a complex toric surfaces. Lens 170 comprises a circular optical portion 172 with an optical axis 174 and a hole 176 coaxial with the axis 174. The lens 170 also comprises a circular non-optical portion 178 having no optical power, within the optical portion 172 and concentric with the optical portion 172.

Figure 17b shows a half of a cross section of lens 170 along a plane A-A parallel to axis 174 shown in Figure 17a. Figure 17b shows a half of a cross section of lens 170 along a plane C-C parallel to axis 174 shown in Figure 17a.

The outer surface of lens 170 follows a first toric surface along plane A-A, and a second toric surface along plane C-C.

As detailed in Figure 17b, optical portion 172 is comprised of a series of concentric circular rings formed on the inner surface of the lens in a stepped arrangement, for example in a similar way as in the embodiment shown in Figures 12a-c.

As shown in figure 17b, which also illustrates how light rays traverse the lens, the first toric surface cooperates with the stepped inner surface of the lens such that the lens 170 has a first focal point 1701 in plane A-A. On the other hand, as illustrated in figure 17c, the second toric surface cooperates with the stepped inner surface of the lens such that the lens 170 has a second focal point 1702 in plane C-C.

According to the present invention, the lenses can be formed of a biocompatible material that permits sufficient gas diffusion to allow adequate oxygenation of internal eye tissues (such materials may include silicone, hydrogels, urethanes or acrylics). Materials which may be used in forming intraocular lenses are generally known in the art, as disclosed, for example, in US Patent No. 5,217,491. Preferably, the lenses according to the present invention are deformable.

It should be understood that the foregoing relates to exemplary embodiments of the invention and that modifications may be made without departing from the scope of the following claims.

For example, each of the anterior and the posterior surfaces of a lens according to the present invention can have at least a portion of any of the following surface types: spherical with a single focus; spherical with two or more focuses; non-spherical with a progressive focus zone; toric; aspheric and plane. Further, each portion of the anterior and the posterior surfaces of a lens according to the present invention can be smooth or stepped.

The radius of curvature of the anterior and the posterior surfaces of a portion of a lens according to an embodiment of the invention can be identical or can be different. Further, a surface of a portion of a lens can have multiple radii of curvature along the perimeter of the section, which may allow the lens to compensate for corneal spherical aberration.

Also, an embodiment of the present invention may comprise an intracorneal lens having an optical portion as described hereabove; and a haptic portion surrounding said optical portion, wherein the haptic portion is corrugated. The intraocular lens may comprise an inner domed portion; and an outer portion having a plurality of tabs disposed peripherally on the outer portion, wherein the domed portion is spaced axially from the plurality of tabs. An embodiment of the present invention may also comprise an intracorneal lens having a central optical portion; and an outer haptic portion, wherein the haptic portion includes an annular portion disposed adjacent to, and radially outward from, the optical portion; a pair of inner arcuate corrugations disposed adjacent to, and radially outward from, the annular portion, the pair of inner arcuate corrugations disposed on opposite sides of the optical portion; and a pair of outer arcuate corrugations disposed adjacent to, and radially outward from, the pair of inner arcuate corrugations. The haptic may comprise at least one irrigation channel radially disposed within said haptic. The arcuate corrugations of the pairs of corrugations may be concentric.

The lenses described hereabove comprise each a single hole. However, embodiments of the present invention may comprise additional holes in other parts of the lens. The additional holes can for example be provided for nutrient transfer but not for alignment, and can have a diameter inferior to the diameter of the central hole. This would render the peripheral holes too small to be seen by one that manipulates the lens, but would help not impairing the optical properties of the lens.

The invention is not to be limited to the embodiments previously described, but is defined by the claims that follow.

## Claims

1. A lens (40, 60, 70, 80, 90, 100, 110, 120, 130, 160, 170) provided for being implanted in a cornea, comprising:
an optical portion (42, 62, 72, 82, 92, 102, 112, 122, 132, 164, 167, 172) having an optical axis (44, 64, 74, 84, 94, 104, 114, 124, 134, 166, 174) passing through the center of the lens (40, 60, 70, 80, 90, 100, 110, 120, 130, 160, 170); and
a hole (46, 66, 76, 86, 96, 106, 116, 126, 136, 168, 176) through the lens 76, concentric with the optical axis (44, 64, 74, 84, 94, 104, 114, 124, 134, 166, 174); **characterized in that** the dimension and shape of the hole (46, 66, 76, 86, 96, 106, 116, 126, 136, 168, 176) are chosen so that the hole (46, 66, 76, 86, 96, 106, 116, 126, 136, 168, 176) has a diameter of larger than 100 micrometer to smaller than 200 micrometer and does not impair the optical properties of the lens, and remains visible to one that manipulates the lens, and allows a flow of nutrients through said hole (46, 66, 76, 86, 96, 106, 116, 126, 136, 168, 176).

2. The lens of claim 1, **characterized in that** the lens comprises at least one circular non-optical portion (68, 78, 88, 98, 108, 118, 128, 138, 178) having no optical power and being concentric with the hole (66, 76, 86, 96, 106, 116, 126, 136, 176).

3. The lens of claim 2, **characterized in that** the non-optical portion (68, 78, 88, 98, 108, 118, 128, 138, 178) is surrounded by the optical portion (62, 72, 82, 92, 102, 112, 122, 132, 172).

4. The lens of any of claim 1 to 3, **characterized in that** the hole (46, 66, 76, 86, 96, 106, 116, 126, 136, 168, 176) is a single hole.

5. The lens of any of claim 1 to 4, **characterized in that** the diameter of the hole (46, 66, 76, 86, 96, 106, 116, 126, 136, 168, 176) varies along the depth of the hole (46, 66, 76, 86, 96, 106, 116, 126, 136, 168, 176).

6. The lens of claim 5, **characterized in that** a first portion of the walls of the hole (116, 126) follows a first portion of a cone, the diameter of the hole (116, 126) decreasing from a first outer diameter, at an entrance of the hole (116, 126), to an inner diameter smaller than the first outer diameter, at an intermediate position within the hole (116, 126), and wherein a second portion of the walls of the hole (116, 126) follows a second portion of a cone, increasing from the inner diameter to a second outer diameter, at the other entrance of the hole (116, 126).

7. The lens of claim 5, **characterized in that** a first portion of the walls of the hole (76, 86, 126) follows a first portion of a torus, the diameter of the hole (76, 86, 126) decreasing from a first outer diameter, at an entrance of the hole (76, 86, 126), to an inner diameter smaller than the first outer diameter, at an intermediate position within the hole (76, 86, 126), and wherein a second portion of the walls of the hole (76, 86, 126) follows a second portion of a torus, increasing from the inner diameter to a second outer diameter, at the other entrance of the hole (76, 86, 126).

8. The lens of claim 5, **characterized in that** a first portion of the walls of the hole (96, 106) follows a portion of a cone, the diameter of the hole (96, 106) decreasing from a first outer diameter, at an entrance of the hole(96, 106), to an inner diameter smaller than the first outer diameter, at an intermediate position within the hole(96, 106), and wherein a second portion of the walls of the hole (96, 106) follows a portion of a torus, increasing from the inner diameter to a second outer diameter, at the other entrance of the hole (96, 106).

9. The lens of any of claims 6 to 8, **characterized in that** a third portion of the walls of the hole (76, 86, 96, 106, 116, 126), between the first and second portions of the walls of the hole (76, 86, 96, 106, 116, 126), follows a cylinder having a diameter equal to the inner diameter.

10. The lens of claim 5, **characterized in that** the walls of the hole (46, 66, 76, 86, 96, 106, 116, 126, 136, 168, 176) follow a cone from one entrance of the hole (46, 66, 76, 86, 96, 106, 116, 126, 136, 168, 176) to the other entrance to the hole (46, 66, 76, 86, 96, 106, 116, 126, 136, 168, 176).

11. The lens of claim 5, **characterized in that** the walls of the hole (46, 66, 76, 86, 96, 106, 116, 126, 136, 168, 176) follow a cylinder from one entrance of the hole (46, 66, 76, 86, 96, 106, 116, 126, 136, 168, 176) to the other entrance of the hole (46, 66, 76, 86, 96, 106, 116, 126, 136, 168, 176).

12. The lens of any of claims 1 to 11, **characterized in that** each of the anterior and the posterior surfaces of the lens comprises at least a portion of one of the following surface types:
spherical surface, with a single focus;
spherical surface, with two or more focuses;
non-spherical surface, with a progressive focus zone;
toric surface; and
flat surface.

13. The lens of any of claims 1 to 12, **characterized in that** at least one of the anterior and the posterior surfaces comprises a stepped portion (1220, 1222, 1224, 1226, 1320, 1322, 1324, 1326).

## Patentansprüche

1. Linse (40, 60, 70, 80, 90, 100, 110, 120, 130, 160, 170), die zur Implantation in eine Kornea vorgesehen ist, aufweisend:
Einen optischen Abschnitt (42, 62, 72, 82, 92, 102, 112, 122, 132, 164, 167, 172) mit einer optischen Achse (44, 64, 74, 84, 94, 104, 114, 124, 134, 166, 174), die durch den Mittelpunkt der Linse (40, 60, 70, 80, 90, 100, 110, 120, 130, 160, 170) geht; und
ein Loch (46, 66, 76, 86, 96, 106, 116, 126, 136, 168, 176) durch die Linse, das mit der optischen Achse (44, 64, 74, 84, 94, 104, 114, 124, 134, 166, 174) konzentrisch ist;
**dadurch gekennzeichnet, dass** die Dimension und Form des Lochs (46, 66, 76, 86, 96, 106, 116, 126, 136, 168, 176) so gewählt sind, dass das Loch (46, 66, 76, 86, 96, 106, 116, 126, 136, 168, 176) einen Durchmesser größer als 100 Mikrometer bis kleiner als 200 Mikrometer hat und die optischen Eigenschaften der Linse nicht beeinträchtigt und für jemanden, der die Linse manipuliert, sichtbar bleibt und einen Nährstoffstrom durch das Loch (46, 66, 76, 86, 96, 106, 116, 126, 136, 168, 176) ermöglicht.

2. Linse nach Anspruch 1, **dadurch gekennzeichnet, dass** die Linse mindestens einen kreisförmigen, nicht optischen Abschnitt (68, 78, 88, 98, 108, 118, 128, 138, 178) aufweist, der keine Brechkraft hat und mit dem Loch (46, 66, 76, 86, 96, 106, 116, 126, 136, 168, 176) konzentrisch ist.

3. Linse nach Anspruch 2, **dadurch gekennzeichnet, dass** der nicht optische Abschnitt (68, 78, 88, 98, 108, 118, 128, 138, 178) von dem optischen Abschnitt (42, 62, 72, 82, 92, 102, 112, 122, 132, 164, 167, 172) umgeben ist.

4. Linse nach einem der Ansprüche 1 bis 3, **dadurch gekennzeichnet, dass** das Loch (46, 66, 76, 86, 96, 106, 116, 126, 136, 168, 176) ein einzelnes Loch ist.

5. Linse nach einem der Ansprüche 1 bis 4, **dadurch gekennzeichnet, dass** der Durchmesser des Lochs (46, 66, 76, 86, 96, 106, 116, 126, 136, 168, 176) entlang der Tiefe des Lochs (46, 66, 76, 86, 96, 106, 116, 126, 136, 168, 176) variiert.

6. Linse nach Anspruch 5, **dadurch gekennzeichnet, dass** ein erster Abschnitt der Wände des Lochs (116, 126) einem ersten Abschnitt eines Kegels folgt, wobei der Durchmesser des Lochs (116, 126) von einem ersten Außendurchmesser an einem Eingang des Lochs (116, 126) an einer Zwischenposition in dem Loch (116, 126) zu einem Innendurchmesser abnimmt, der kleiner als der erste Außendurchmesser ist, und wobei ein zweiter Abschnitt der Wände des Lochs (116, 126) einem zweiten Abschnitt eines Kegels folgt und vom Innendurchmesser zu einem zweiten Außendurchmesser am anderen Eingang des Lochs (116, 126) zunimmt.

7. Linse nach Anspruch 5, **dadurch gekennzeichnet, dass** ein erster Abschnitt der Wände des Lochs (76, 86, 126) einem ersten Abschnitt eines Torus folgt, wobei der Durchmesser des Lochs (76, 86, 126) von einem ersten Außendurchmesser an einem Eingang des Lochs (76, 86, 126) an einer Zwischenposition in dem Loch (76, 86, 126) zu einem Innendurchmesser abnimmt, der kleiner als der erste Außendurchmesser ist, und wobei ein zweiter Abschnitt der Wände des Lochs (76, 86, 126) einem zweiten Abschnitt eines Torus folgt und vom Innendurchmesser zu einem zweiten Außendurchmesser am anderen Eingang des Lochs (76, 86, 126) zunimmt.

8. Linse nach Anspruch 5, **dadurch gekennzeichnet, dass** ein erster Abschnitt der Wände des Lochs (96, 106) einem Abschnitt eines Kegels folgt, wobei der Durchmesser des Lochs (96, 106) von einem ersten Außendurchmesser an einem Eingang des Lochs (96, 106) an einer Zwischenposition in dem Loch (96, 106) zu einem Innendurchmesser abnimmt, der kleiner als der erste Außendurchmesser ist, und wobei ein zweiter Abschnitt der Wände des Lochs (96, 106) einem Abschnitt eines Torus folgt und vom Innendurchmesser zu einem zweiten Außendurchmesser am anderen Eingang des Lochs (96, 106) zunimmt.

9. Linse nach einem der Ansprüche 6 bis 8, dadurch gekennzeichet, dass ein dritter Abschnitt der Wände des Lochs (76, 86, 96, 106, 116, 126) zwischen dem ersten und zweiten Abschnitt der Wände des Lochs (76, 86, 96, 106, 116, 126) einem Zylinder mit einem Durchmesser gleich dem Innendurchmesser folgt.

10. Linse nach Anspruch 5, **dadurch gekennzeichnet, dass** die Wände des Lochs (46, 66, 76, 86, 96, 106, 116, 126, 136, 168, 176) einem Kegel von einem Eingang des Lochs (46, 66, 76, 86, 96, 106, 116, 126, 136, 168, 176) zum anderen Eingang des Lochs (46, 66, 76, 86, 96, 106, 116, 126, 136, 168, 176) folgen.

11. Linse nach Anspruch 5, **dadurch gekennzeichnet, dass** die Wände des Lochs (46, 66, 76, 86, 96, 106, 116, 126, 136, 168, 176) einem Zylinder von einem Eingang des Lochs (46, 66, 76, 86, 96, 106, 116, 126, 136, 168, 176) zum anderen Eingang des Lochs (46, 66, 76, 86, 96, 106, 116, 126, 136, 168, 176) folgen.

12. Linse nach einem der Ansprüche 1 bis 11, **dadurch gekennzeichnet, dass** sowohl die vordere wie auch hintere Oberfläche der Linse mindestens einen Abschnitt einer der folgenden Oberflächenarten aufweisen:
sphärische Oberfläche mit einem einzigen Brennpunkt;
sphärische Oberfläche mit zwei oder mehr Brennpunkten;
nicht sphärische Oberfläche mit einer progressiven Brennpunktzone;
torische Oberfläche; und
flache Oberfläche.

13. Linse nach einem der Ansprüche 1 bis 12, **dadurch gekennzeichnet, dass** mindestens eine der vorderen und hinteren Oberfläche einen abgestuften Abschnitt (1220, 1222, 1224, 1226, 1320, 1322, 1324, 1326) aufweist.

## Revendications

1. Lentille (40, 60, 70, 80, 90, 100, 110, 120, 130, 160, 170) fournie pour être implantée dans une cornée, comprenant :
une partie optique (42, 62, 72, 82, 92, 102, 112, 122, 132, 164, 167, 172) ayant un axe optique (44, 64, 74, 84, 94, 104, 114, 124, 134, 166, 174) passant par le centre de la lentille (40, 60, 70, 80, 90, 100, 110, 120, 130, 160, 170) ; et
un trou (46, 66, 76, 86, 96, 106, 116, 126, 136, 168, 176) à travers la lentille concentrique avec l'axe optique (44, 64, 74, 84, 94, 104, 114, 124, 134, 166, 174) ; **caractérisée en ce que** la dimension et la forme du trou (46, 66, 76, 86, 96, 106, 116, 126, 136, 168, 176) sont choisies de telle sorte que le trou (46, 66, 76, 86, 96, 106, 116, 126, 136, 168, 176) a un diamètre allant de plus de 100 micromètres à moins de 200 micromètres et ne détériore pas les propriétés optiques de la lentille, reste visible à une personne qui manipule la lentille, et permet une circulation de nutriments à travers ledit trou (46, 66, 76, 86, 96, 106, 116, 126, 136, 168, 176).

2. Lentille selon la revendication 1, **caractérisée en ce que** la lentille comprend au moins une partie non optique circulaire (68, 78, 88, 98, 108, 118, 128, 138, 178) n'ayant aucune puissance optique et étant concentrique avec le trou (66, 76, 86, 96, 106, 116, 126, 136, 176).

3. Lentille selon la revendication 2, **caractérisée en ce que** la partie non optique (68, 78, 88, 98, 108, 118, 128, 138, 178) est entourée par la partie optique (62, 72, 82, 92, 102, 112, 122, 132, 172).

4. Lentille selon l'une quelconque des revendications 1 à 3, **caractérisée en ce que** le trou (46, 66, 76, 86, 96, 106, 116, 126, 136, 168, 176) est un trou unique.

5. Lentille selon l'une quelconque des revendications 1 à 4, **caractérisée en ce que** le diamètre du trou (46, 66, 76, 86, 96, 106, 116, 126, 136, 168, 176) varie sur la profondeur du trou (46, 66, 76, 86, 96, 106, 116, 126, 136, 168, 176).

6. Lentille selon la revendication 5, **caractérisée en ce qu'**une première partie des parois du trou (116, 126) suit une première partie d'un cône, le diamètre du trou (116, 126) diminuant depuis un premier diamètre extérieur, à une entrée du trou (116, 126), jusqu'à un diamètre intérieur plus petit que le premier diamètre extérieur, à une position intermédiaire à l'intérieur du trou (116, 126), et dans laquelle une deuxième partie des parois du trou (116, 126) suit une deuxième partie d'un cône, augmentant depuis le diamètre intérieur jusqu'à un deuxième diamètre extérieur, à l'autre entrée du trou (116, 126).

7. Lentille selon la revendication 5, **caractérisée en ce qu'**une première partie des parois du trou (76, 86, 126) suit une première partie d'un tore, le diamètre du trou (76, 86, 126) diminuant depuis un premier diamètre extérieur, à une entrée du trou (76, 86, 126), jusqu'à un diamètre intérieur plus petit que le premier diamètre extérieur, à une position intermédiaire à l'intérieur du trou (76, 86, 126), et dans laquelle une deuxième partie des parois du trou (76, 86, 126) suit une deuxième partie d'un tore, augmentant depuis le diamètre intérieur jusqu'à un deuxième diamètre extérieur, à l'autre entrée du trou (76, 86, 126).

8. Lentille selon la revendication 5, **caractérisée en ce qu'**une première partie des parois du trou (96, 106) suit une partie d'un cône, le diamètre du trou (96, 106) diminuant depuis un premier diamètre extérieur, à une entrée du trou (96, 106), jusqu'à un diamètre intérieur plus petit que le premier diamètre extérieur, à une position intermédiaire à l'intérieur du trou (96, 106), et dans laquelle une deuxième partie des parois du trou (96, 106) suit une partie d'un tore, augmentant depuis le diamètre intérieur jusqu'à un deuxième diamètre extérieur, à l'autre entrée du trou (96, 106).

9. Lentille selon l'une quelconque des revendications 6 à 8, **caractérisée en ce qu'**une troisième partie des parois du trou (76, 86, 96, 106, 116, 126), entre la première et la deuxième partie des parois du trou (76, 86, 96, 106, 116, 126), suit un cylindre ayant un diamètre égal au diamètre intérieur.

10. Lentille selon la revendication 5, **caractérisée en ce que** les parois du trou (46, 66, 76, 86, 96, 106, 116, 126, 136, 168, 176) suivent un cône depuis une entrée du trou (46, 66, 76, 86, 96, 106, 116, 126, 136, 168, 176) jusqu'à l'autre entrée du trou (46, 66, 76, 86, 96, 106, 116, 126, 136, 168, 176).

11. Lentille selon la revendication 5, **caractérisée en ce que** les parois du trou (46, 66, 76, 86, 96, 106, 116, 126, 136, 168, 176) suivent un cylindre depuis une entrée du trou (46, 66, 76, 86, 96, 106, 116, 126, 136, 168, 176) jusqu'à l'autre entrée du trou (46, 66, 76, 86, 96, 106, 116, 126, 136, 168, 176).

12. Lentille selon l'une quelconque des revendications 1 à 11, **caractérisée en ce que** chacune des surfaces antérieure et postérieure de la lentille comprend au moins une partie de l'un des types de surface suivants :
surface sphérique, avec un seul foyer;
surface sphérique, avec au moins deux foyers;
surface non sphérique, avec une zone focale progressive ;
surface torique ; et
surface plate.

13. Lentille selon l'une quelconque des revendications 1 à 12, **caractérisée en ce que** l'une au moins des surfaces antérieure et postérieure comprend une partie en gradins (1220, 1222, 1224, 1226, 1320, 1322, 1324, 1326).
